# EUROPÄISCHE PATENTSCHRIFT

(11) **EP 0 876 341 B1**
(45) Veröffentlichungstag und Bekanntmachung des Hinweises auf die Patenterteilung: **09.08.2000**
(21) Anmeldenummer: 96943913.2
(22) Anmeldetag: 11.12.1996
(51) Int. Cl.: C07D 201/08, C07C 209/48

(54) **VERFAHREN ZUR GLEICHZEITIGEN HERSTELLUNG VON CAPROLACTAM UND HEXAMETHYLENDIAMIN**
PROCESS FOR SIMULTANEOUSLY PREPARING CAPROLACTAM AND HEXAMETHYLENE DIAMINE
PROCEDE DE PRODUCTION SIMULTANEE DE CAPROLACTAME ET DE DIAMINE D'HEXAMETHYLENE

(30) Priorität: 22.12.1995 DE 19548289
(43) Veröffentlichungstag der Anmeldung: 11.11.1998
(73) Patentinhaber: BASF AKTIENGESELLSCHAFT, 67056 Ludwigshafen (DE)
(72) Erfinder: ACHHAMMER, Günther, D-68309 Mannheim (DE); BASSLER, Peter, D-68519 Viernheim (DE); FISCHER, Rolf, D-69121 Heidelberg (DE); FUCHS, Eberhard, D-67227 Frankenthal (DE); LUYKEN, Hermann, D-67069 Ludwigshafen (DE); SCHNURR, Werner, D-67273 Herxheim (DE); VOIT, Guido, D-69198 Schriesheim (DE); HILPRECHT, Lutz, D-67063 Ludwigshafen (DE)
(86) Internationale Anmeldenummer: EP9605521
(87) Internationale Veröffentlichungsnummer: WO9723454

(56) Entgegenhaltungen:
- WO-A-92/21650
- US-A- 2 257 814
- J. CHEM. SOC. FARADAY TRANS., Bd. 89, Nr. 18, 1993, Seiten 3507-3512, XP000387156 F. MEDINA ET AL.: "Surface Characterization and Hydrogenation Properties of Several Nickel/alpha-Alumina Catalysts"
- CHEMICAL ABSTRACTS, vol. 82, no. 10, 10.März 1975 Columbus, Ohio, US; abstract no. 58479x, Seite 29; XP002029916 & JP 74 019 250 A (TEIJIN LTD.) 16.Mai 1974

## Beschreibung

Die vorliegende Erfindung betrifft ein Verfahren zur gleichzeitigen Herstellung von Caprolactam und Hexamethylendiamin ausgehend von Adipodinitril.

Des weiteren betrifft die Erfindung ein verbessertes Verfahren zur gleichzeitigen Trennung von 6-Aminocapronitril und Hexamethylendiamin aus einer diese Stoffe enthaltenden Mischung.

Die partielle Hydrierung von Adipodinitril zu 6-Aminocapronitril in Gegenwart von Ammoniak und verschiedenen Katalysatoren ist hinreichend beschrieben worden. So beschreibt die US 4 601 859 die Verwendung von Katalysatoren auf der Basis von Rhodium auf Magnesiumoxid, die US 2 762 835 beschreibt die Verwendung von Raney-Nickel, die US 2 208 598 beschreibt die Verwendung von Nickel auf Aluminiumoxid, die DE-A 848 654 beschreibt Festbettkatalysatoren auf der Basis von Kupfer/Cobalt/Zink- und Eisen/Cobalt-Spinellen, die DE-A 954 416 beschreibt die Verwendung von Cobalt auf Kieselgel und die DE-A 4 235 466 beschreibt die Verwendung von Eisenschwämmen.

Nach dem in der WO 92/21650 beschriebenen Verfahren werden in Gegenwart von Raney-Nickel Aminocapronitril-Ausbeuten von 60% bei einem Umsatz von 70% und Hexamethylendiamin-Ausbeuten von 9% erzielt. Bei einem Umsatz von 80% beträgt die Ausbeute 62%.

Es ist weiterhin bekannt, 6-Aminocapronitril mit Wasser in der Gas- oder Flüssigphase, in Gegenwart oder Abwesenheit von Katalysatoren unter Freisetzung von Ammoniak zu Caprolactam umzusetzen. So beschreibt die US 2 301 964 ein Verfahren, in dem 10 bis 25%ige Lösungen von 6-Aminocapronitril in der Flüssigphase auf 250 bis 290°C zu Caprolactam mit Ausbeuten bis zu 76% umgesetzt werden.

Weiterhin ist die Cyclisierung von 25- bis 35%igen 6-Aminocapronitril-Lösungen bei 220°C in der Flüssigphase in Wasser unter Zusatz organischer Lösungsmittel in Gegenwart von Zink-, Kupfer-, Blei- und Quecksilberverbindungen in der FR-A 2,029,540 beschrieben. Hierbei erzielt man Caprolactam-Ausbeuten von bis zu 83%.

Die Cyclisierung von 6-Aminocapronitril läßt sich auch in der Gasphase durchführen (US 2 357 484): ausgehend von 80%igen wäßrigen Lösungen erzielt man bei 305°C mit Aluminiumoxid als Katalysator Caprolactam-Ausbeuten von 92%.

Die EP-A 150 295 beschreibt die Umsetzung von 6-Aminocapronitril in der Gasphase in Gegenwart von Kupfer-Vanadin-Katalysatoren, Wasserstoff, Wasser und Ammoniak bei 290°C mit 77% Ausbeute an Caprolactam.

Des weiteren beschreibt die DE-A 43 19 134 die Umsetzung von 6-Aminocapronsäurenitril in Wasser in flüssiger Phase ohne Zusatz eines Katalysators zu Caprolactam.

Ein Verfahren wie man ausgehend von Adipodinitril über 6-Aminocapronitril zu Caprolactam in einem beide Verfahrensschritte zusammenfassenden Gesamtprozeß gelangt, läßt sich den zuvor genannten Dokumenten nicht entnehmen.

Aufgabe der vorliegenden Erfindung war es daher, ein Verfahren zur gleichzeitigen Herstellung von Caprolactam und Hexamethylendiamin ausgehend von Adipodinitril zur Verfügung zu stellen. Des weiteren sollte ein Verfahren zur Verfügung gestellt werden, welches aus dem bei der partiellen Hydrierung von Adipodinitril erhaltenen Reaktionsgemisch in einem kontinuierlichen Prozeß reines 6-Aminocapronitril und Hexamethylendiamin liefert, wobei das 6-Aminocapronitril in einem weiteren Verfahrensschritt zu Caprolactam cyclisiert wird. Ferner sollten bei diesem Verfahren erhaltene Nebenprodukte so weit wie möglich wiederverwertet, bevorzugt in eine frühere Verfahrensstufe rückgeführt, werden.

Demgemäß wurde ein Verfahren zur gleichzeitigen Herstellung von Caprolactam und Hexamethylendiamin ausgehend von Adipodinitril gefunden, indem man
(a) Adipodinitril partiell hydriert unter Erhalt einer Mischung, enthaltend im wesentlichen 6-Aminocapronitril, Hexamethylendiamin, Ammoniak, Adipodinitril und Hexamethylenimin, und
(b) die in (a) erhaltene Mischung einer Destillation unter Erhalt von Ammoniak als Kopfprodukt und eines Sumpfes I unterwirft, wobei man die Destillation bei einer Sumpftemperatur im Bereich von 60 bis 250°C und einem Druck im Bereich von 5 bis 30 bar in Gegenwart von einer unter den Destillationsbedingungen inerten Verbindung A oder einem Gemisch von inerten Verbindungen A durchführt, die bei einem Druck von 18 bar bei einer Temperatur im Bereich von 60 bis 250°C siedet, und wobei man den Ammoniak nicht vollständig abtrennt, und
(c) den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber demjenigen aus der Mischung, die in Stufe (b) eingesetzt wird, einer zweiten Destillation unterwirft unter Erhalt einer Mischung aus der inerten Verbindung A und Ammoniak als Kopfprodukt und eines Sumpfes II, wobei man die Destillation bei einer Sumpftemperatur im Bereich von 100 bis 250°C, und einem Druck im Bereich von 2 bis 15 bar durchführt, mit der Maßgabe, daß man die Drücke der ersten und der zweiten Kolonne so aufeinander abstimmt, daß man bei einer Sumpftemperatur von jeweils maximal 250°C eine Kopftemperatur von über 20°C erhält, oder in deren Kondensator
   die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
   den Kopfabzug der zweiten Kolonne dampfförmig nach Druckerhöhung mit einem Verdichter in die erste Kolonne oder in deren Kondensator zurückführt,
(d) den Sumpf II, enthaltend im wesentlichen 6-Aminocapronitril, Hexamethylendiamin, Adipodinitril, Hexamethylenimin und inerte Verbindung(en) A in einer dritten Kolonne einer Destillation unterwirft unter Erhalt der inerten Verbindung(en) A als Kopfprodukt und eines Sumpfes III, wobei man die Destillation bei einer Sumpftemperatur im Bereich von 50 bis 250°C und einem Druck im Bereich von 0,05 bis 2 bar durchführt, mit der Maßgabe, daß man die als Kopfprodukt erhaltenen inerten Verbindungen A der zweiten Kolonne zuführt, und gewünschtenfalls die Destillation in Gegenwart von einer oder mehreren unter den Destillationsbedingungen inerten Verbindung(en) B durchführt, die bei einem Druck von 0,3 bar im Bereich von 20 bis 250°C sieden,
(e) den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne einer Destillation unterwirft unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und der Druck im Bereich von 0,05 bis 1,5 bar liegen unter Erhalt eines Sumpfes IV, und gewünschtenfalls die Kolonne mit einer Trennwand im Bereich zwischen Zulauf und Seitenabzug ausstattet (Petyuk-Kolonne), so daß das gewonnene Hexamethylendiamin im wesentlichen frei ist von Hexamethylenimin und inerten Verbindungen(en) B sowie von anderen Leichtsiedern, wobei
   man Kopfprodukt KP1 der dritten Kolonne zuführt oder gegebenenfalls nur teilweise der dritten Kolonne zuführt und den Rest ausschleust, und
(f) den Sumpf IV, enthaltend im wesentlichen 6-Aminocapronitril und Adiponitril sowie eventuelle Hochsieder in einer fünften Kolonne einer Destillation unterwirft, unter Erhalt von 6-Aminocapronitril mit einer Reinheit von mindestens 95 % als Kopfprodukt und eines Seitenabzuges, bestehend im wesentlichen aus Adipodinitril, sowie eines Sumpfes V, das aus Hochsiedern und geringen Mengen Adipodinitril besteht, und
   gewünschtenfalls die Kolonne mit einer Trennwand im Bereich zwischen Zulauf und Seitenabzug ausstattet, so daß das gewonnene Adipodinitril niedrigere Anteile an Hochsiedern enthält, wobei
   die Destillation bei einer Sumpftemperatur im Bereich von 50 bis 250°C und einem Druck im Bereich von 10 bis 300 mbar durchgeführt wird,
und das so erhaltene 6-Aminocapronitril anschließend zu Caprolactam cyclisiert.

Des weiteren wurde ein Verfahren zur gleichzeitigen Trennung 6-Aminocapronitril und Hexamethylendiamin aus einer diese Stoffe enthaltenden Mischung gefunden.

Die partielle Hydrierung von Adipodinitril kann man nach einem der bekannten Verfahren durchführen, beispielsweise nach einem der zuvor genannten Verfahren beschrieben in US 4 601 859, US 2 762 835, US 2 208 598, DE-A 848 654, DE-A 954 416, DE-A 4 235 466 oder WO 92/21650, indem man im allgemeinen die Hydrierung in Gegenwart von Nickel-, Cobalt-, Eisen- oder Rhodium-haltigen Katalysatoren durchführt. Dabei können die Katalysatoren als Trägerkatalysatoren oder als Vollkatalysatoren verwendet werden. Als Katalysatorträger kommen beispielsweise Aluminiumoxid, Siliciumdioxid, Titandioxid, Magnesiumoxid, Aktivkohlen und Spinelle in Frage. Als Vollkatalysatoren kommen beispielsweise Raney-Nickel und Raney-Cobalt in Betracht.

Üblicherweise wählt man die Katalysatorbelastung im Bereich von 0,05 bis 10, vorzugsweise von 0,1 bis 5 kg Adipodinitril/ l_{Katalysator}*h.

Die Hydrierung nimmt man in der Regel bei Temperaturen im Bereich von 20 bis 200, vorzugsweise von 50 bis 150°C, und bei Wasserstoff-Partialdrücken von 0,1 bis 20, vorzugsweise von 0,5 bis 10 MPa, vor.

Bevorzugt führt man die Hydrierung in Gegenwart eines Lösungsmittels, insbesondere Ammoniak, durch. Die Ammoniak-Menge wählt man im allgemeinen im Bereich von 0,1 bis 10, vorzugsweise von 0,5 bis 3 kg Ammoniak/kg Adipodinitril.

Das Molverhältnis von 6-Aminocapronitril zu Hexamethylendiamin, und damit das Molverhältnis von Caprolactam zu Hexamethylendiamin, kann durch den jeweils gewählten Adipodinitril-Umsatz gesteuert werden. Bevorzugt arbeitet man bei Adipodinitril-Umsätzen im Bereich von 10 bis 90, bevorzugt von 30 bis 80%, um hohe 6-Aminocapronitril-Selektivitäten zu erhalten.

In der Regel liegt die Summe aus 6-Aminocapronitril und Hexamethylendiamin je nach Katalysator und Reaktionsbedingungen bei 95 bis 99%, wobei als mengenmäßig bedeutendstes Nebenprodukt Hexamethylenimin auftritt.

In einer bevorzugten Ausführungsform nimmt man die Umsetzung in Gegenwart von Ammoniak und Lithiumhydroxid, oder einer Lithiumverbindung, die unter den Reaktionsbedingungen Lithiumhydroxid bildet, bei Temperaturen im Bereich von 40 bis 120, vorzugsweise von 50 bis 100, besonders vorzugsweise von 60 bis 90°C vor; den Druck wählt man im allgemeinen im Bereich von 2 bis 12, vorzugsweise von 3 bis 10, besonders bevorzugt von 4 bis 8 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bevorzugt wählt man die Druck- und Temperaturbereiche so, daß man die Umsetzung in flüssiger Phase vornehmen kann.

Ammoniak setzt man im allgemeinen in einer Menge ein, so daß das Gewichtsverhältnis von Ammoniak zu Dinitril im Bereich von 9:1 bis 0,1:1, bevorzugt von 2,3:1 bis 0,25:1, besonders bevorzugt von 1,5:1 bis 0,4:1, liegt.

Die Menge an Lithiumhydroxid wählt man in der Regel im Bereich von 0,1 bis 20, bevorzugt von 1 bis 10 Gew.-%, bezogen auf die Menge an eingesetztem Katalysator.

Als Lithiumverbindungen, die unter den Reaktionsbedingungen Lithiumhydroxid bilden, seien genannt: Lithiummetall, Alkyl- und Aryllithiumverbindungen wie n-Butyllithium und Phenyllithium. Die Menge an diesen Verbindungen wählt man im allgemeinen so, daß die zuvor genannte Menge an Lithiumhydroxid erhalten wird.

Als Katalysatoren setzt man bevorzugt Nickel-, Ruthenium-, Rhodium-, Eisen- und Cobalt-haltige Verbindungen ein, bevorzugt solche vom Raney-Typ, insbesondere Raney-Nickel und Raney-Cobalt. Man kann die Katalysatoren auch als Trägerkatalysatoren einsetzen, wobei als Träger beispielsweise Aluminiumoxid, Siliciumdioxid, Zinkoxid, Aktivkohle oder Titandioxid dienen können. (s. Appl. Het. Cat., 1987, S. 106-122; Catalysis, Vol. 4 (1981) S. 1-30). Besonders bevorzugt ist Raney-Nickel (beispielsweise von BASF AG, Degussa und Grace).

Die Nickel-, Ruthenium-, Rhodium-, Eisen- und Cobalt-Katalysatoren können mit Metallen der Gruppe VIB (Cr, Mo, W) und VIII (Fe, Ru, Os, Co (nur im Falle von Nickel), Rh, Ir, Pd, Pt) des Periodensystems modifiziert sein. Nach bisherigen Beobachtungen führt der Einsatz von insbesondere modifizierten Raney-Nickel-Katalysatoren, beispielsweise mit Chrom und/oder Eisen modifiziert, zu höheren Aminonitril-Selektivitäten. (Herstellung siehe DE-A 2 260 978; Bull. Soc. Chem. 13 (1946) S. 208).

Die Menge an Katalysator wählt man im allgemeinen so, daß die Cobalt-, Ruthenium-, Rhodium-, Eisen- oder Nickel-Menge im Bereich von 1 bis 50, bevorzugt von 5 bis 20 Gew.-%, bezogen auf die eingesetzte Menge an Dinitril, beträgt.

Die Katalysatoren können als Festbettkatalysatoren in Sumpf- oder Rieselfahrweise oder als Suspensionskatalysatoren eingesetzt werden.

In einer weiteren bevorzugten Ausführungsform hydriert man Adipodinitril partiell zu 6-Aminocapronitril bei erhöhter Temperatur und erhöhtem Druck in Gegenwart eines Lösungsmittels und eines Katalysators, indem man einen Katalysator verwendet, der
(a) eine Verbindung auf der Basis eines Metalles, ausgewählt aus der Gruppe, bestehend aus Nickel, Cobalt, Eisen, Ruthenium und Rhodium, enthält und
(b) von 0,01 bis 25, vorzugsweise von 0,1 bis 5 Gew.-%, bezogen auf (a), eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
(c) von 0 bis 5, vorzugsweise von 0,1 bis 3 Gew.-%, bezogen auf (a), einer Verbindung auf der Basis eines Alkalimetalles oder eines Erdalkalimetalles, enthält,
mit der Maßgabe, daß, wenn als Komponente (a) eine Verbindung auf der Basis von nur Ruthenium oder Rhodium oder Ruthenium und Rhodium oder Nickel und Rhodium gewählt wird, der Promotor (b) gewünschtenfalls entfallen kann.

Bevorzugte Katalysatoren sind solche, in denen die Komponente (a) mindestens eine Verbindung auf der Basis eines Metalles, ausgewählt aus der Gruppe aus Nickel, Cobalt und Eisen, in einer Menge im Bereich von 10 bis 95 Gew.-% sowie Ruthenium und/oder Rhodium in einer Menge im Bereich von 0,1 bis 5 Gew.-%, jeweils bezogen auf die Summe der Komponenten (a) bis (c), enthält,
die Komponente (b) mindestens einen Promotor auf der Basis eines Metalles, ausgewählt aus der Gruppe bestehend aus Silber, Kupfer, Mangan, Rhenium, Blei und Phosphor, in einer Menge im Bereich von 0,1 bis 5 Gew.-%, bezogen auf (a), enthält, und
die Komponente (c) mindestens eine Verbindung auf der Basis der Alkalimetalle und Erdalkalimetalle, ausgewählt aus der Gruppe bestehend aus Lithium, Natrium, Kalium, Cäsium, Magnesium und Calcium, in einer Menge im Bereich von 0,1 bis 5 Gew.-% enthält.

Besonders bevorzugte Katalysatoren sind:
Katalysator A, enthaltend 90 Gew.-% Cobaltoxid (CoO), 5 Gew.-% Manganoxid (Mn₂O₃), 3 Gew.-% Phosphorpentoxid und 2 Gew.-% Natriumoxid (Na₂O),
Katalysator B, enthaltend 20 Gew.-% Cobaltoxid (CoO), 5 Gew.-% Manganoxid (Mn₂O₃), 0,3 Gew.-% Silberoxid (Ag₂O), 70 Gew.-% Siliciumdioxid (SiO₂), 3,5 Gew.-% Aluminiumoxid (Al₂O₃), 0,4 Gew.-% Eisenoxid (Fe₂O₃), 0,4 Gew.-% Magnesiumoxid (MgO) sowie 0,4 Gew.-% Calciumoxid (CaO), und
Katalysator C, enthaltend 20 Gew.-% Nickeloxid (NiO), 67,42 Gew.-% Siliciumdioxid (SiO₂), 3,7 Gew.-% Aluminiumoxid (Al₂O₃), 0,8 Gew.-% Eisenoxid (Fe₂O₃), 0,76 Gew.-% Magnesiumoxid (MgO), 1,92 Gew.-% Calciumoxid (CaO), 3,4 Gew.-% Natriumoxid (Na₂O) sowie 2,0 Gew.-% Kaliumoxid (K₂O).

Bei den bevorzugt einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man Vorläufer der Komponenten (a) zusammen mit Vorläufern der Promotoren (Komponenten (b) und gewünschtenfalls mit Vorläufern der Spurenkomponenten (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), (b) und gewünschtenfalls (c) auf den Träger nach an sich bekannten Methoden aufsprüht.

Als Vorläufer der Komponenten (a) kommen in der Regel gut wasser lösliche Salze der zuvor genannten Metalle wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle wie Nitrate, Chloride, Acetate, Formiate und Sulfate sowie insbesondere Hexachloroplatinat in Betracht, vorzugsweise Nitrate und Hexachloroplatinat.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man üblicherweise bei Temperaturen im Bereich von 150 bis 500, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 80 bis 250, vorzugsweise von 80 bis 180°C bei Katalysatoren auf der Basis von Ruthenium oder Rhodium als Komponente (a), oder im Bereich von 200 bis 500, vorzugsweise von 250 bis 400°C bei Katalysatoren auf der Basis eines der Metalle ausgewählt aus der Gruppe aus Nickel, Cobalt und Eisen als Komponente (a) 2 bis 24 h einer Wasserstoff-Atmosphäre oder einer Gasmischung, enthaltend Wasserstoff und ein Inertgas wie Stickstoff, aussetzt. Die Katalysatorbelastung beträgt hierbei bevorzugt 200 l pro l Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500, vorzugsweise von 200 bis 350°C in einer Wasserstoff-haltigen Atmosphäre vor.

Die Katalysatoren können als Festbettkatalysatoren in Sumpf- oder Rieselfahrweise oder als Suspensionskatalysatoren eingesetzt werden.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 20, vorzugsweise von 3 bis 10, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 90°C und einen Druck in der Regel im Bereich von 2 bis 30, vorzugsweise von 3 bis 20 MPa wählt. Bevorzugt führt man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/1*h. Auch hier kann man durch Veränderung der Verweilzeit den Umsatz und damit die Selektivität gezielt einstellen.

Die partielle Hydrierung kann man in einem üblichen hierfür geeigneten Reaktor (R1 in Zeichnung) durchführen.

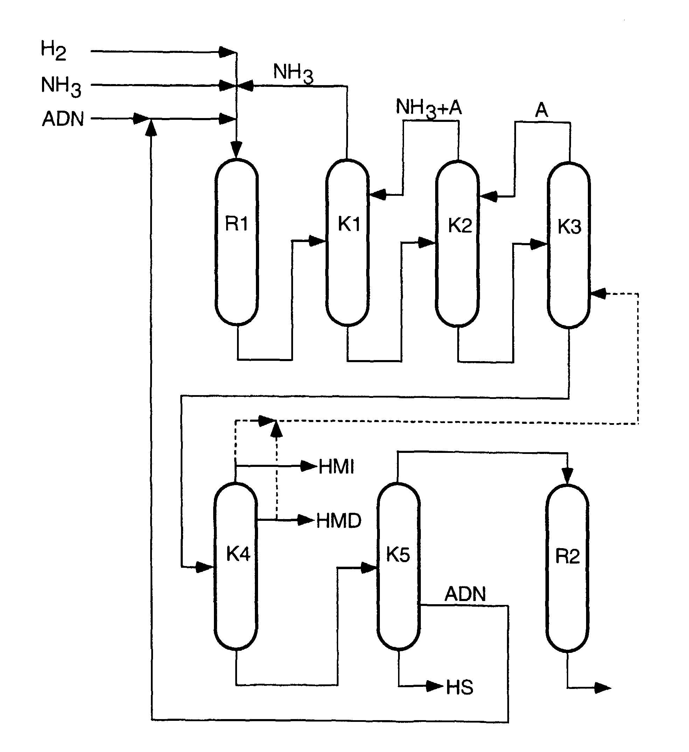

Die Destillation in der ersten Kolonne (Stufe (b); K1 in der Zeichnung) führt man erfindungsgemäß so durch, daß man die Mischung aus Stufe (a), enthaltend im wesentlichen 6-Aminocapronitril, Hexamethylendiamin, Ammoniak, Adipodinitril und Hexamethylenimin, bevorzugt eine Mischung, enthaltend im wesentlichen von 1 bis 70, vorzugsweise von 5 bis 40 Gew.-% 6-Aminocapronitril,
- von: 1 bis 70, vorzugsweise von 5 bis 40 Gew.-% Adipodinitril,
- von: 0,1 bis 70, vorzugsweise von 1 bis 40 Gew.-% Hexamethylendiamin,
- von: 0,01 bis 10, vorzugsweise von 0,05 bis 5 Gew.-% Hexamethylenimin und
- von: 5 bis 95, vorzugsweise von 20 bis 85 Gew.-% Ammoniak, in der Regel in einer üblichen Destillationskolonne, bei einer Sumpftemperatur im Bereich von 60 bis 250, vorzugsweise von 100 bis 200°C und einem Druck im Bereich von 5 bis 30, vorzugsweise von 12 bis 25 bar in Gegenwart von einer oder mehreren unter den Destillationsbedingungen inerten Verbindungen A durchführt, die bei einem Druck von 18 bar bei einer Temperatur im Bereich von 60 bis 220°C sieden, unter Erhalt von Ammoniak als Kopfprodukt und eines Sumpfes I, wobei man den Ammoniak nicht vollständig abtrennt.

Als Verbindung A kommen erfindungsgemäß Substanzen in Betracht, die unter den Destillationsbedingungen inert sind und einen Siedepunkt im Bereich von 60 bis 250, vorzugsweise von 60 bis 150°C bei einem Druck von 18 bar aufweisen. Beispielhaft seien genannt: Alkane, Cycloalkane, Aromaten, Naphthene, Alkohole, Ether, Nitrile und Amine mit den zuvor genannten Eigenschaften, insbesondere C₅-C₈-Alkane und C₂-C₄-Alkanole, besonders bevorzugt n-Pentan, Cyclohexan, Triethylamin, Ethanol, Acetonitril, n-Hexan, Di-n-propylether, Isopropanol, n-Butylamin, Benzol, ganz besonders bevorzugt Ethanol.

Üblicherweise gibt man Verbindung A in einer Menge im Bereich von 0,1 bis 50, vorzugsweise von 1 bis 10 Gew.-%, bezogen auf den Sumpf I, zu.

In Stufe (c) unterwirft man den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber demjenigen aus der Mischung, die in Stufe (b) aus Stufe (a) kommend eingesetzt wird, einer zweiten Destillation unter Erhalt einer Mischung aus der(n) inerten Verbindung(en) A und Ammoniak als Kopfprodukt und eines Sumpfes II, wobei man die Destillation bei einer Sumpftemperatur im Bereich von 100 bis 250, vorzugsweise von 140 bis 200°C, und einem Druck im Bereich von 2 bis 15, vorzugsweise von 4 bis 12 bar durchführt, mit der Maßgabe, daß man die Drücke der ersten und der zweiten Kolonne (K2 in der Zeichnung) so aufeinander abstimmt, daß man bei einer jeweiligen Sumpftemperatur von maximal 250°C eine Kopftemperatur von über 20°C erhält. Es kann auch vorteilhaft sein, daß man die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
den Kopfabzug der zweiten Kolonne dampfförmig nach Druckerhöhung mit einem Verdichter in die erste Kolonne oder in deren Kondensator zurückführt.

In Stufe (d) unterwirft man den Sumpf II, enthaltend im wesentlichen 6-Aminocapronitril, Hexamethylendiamin, Adipodinitril, Hexamethylenimin und inerte Verbindung(en) A in einer dritten Kolonne (K3 in der Zeichnung) einer Destillation unter Erhalt der inerten Verbindung(en) A als Kopfprodukt und eines Sumpfes III, wobei man die Destillation bei einer Sumpftemperatur im Bereich von 50 bis 250, vorzugsweise von 140 bis 200°C und einem Druck im Bereich von 0,05 bis 2, vorzugsweise von 0,2 bis 1 bar durchführt, mit der Maßgabe, daß man die als Kopfprodukt erhaltene(n) inerte(n) Verbindung(en) A der zweiten Kolonne zuführt, und gewünschtenfalls die Destillation in Gegenwart von einer oder mehreren unter den Destillationsbedingungen inerten Verbindung(en) B durchführt, die bei einem gegebenen Druck von 0,3 bar im Bereich von 20 bis 250, vorzugsweise von 60 bis 170°C sieden.

Als Verbindung B seien beispielhaft genannt:
Alkane, Cycloalkane, Aromaten, Naphthene, Alkohole, Ether, Nitrile und Amine mit den zuvor genannten Eigenschaften, insbesondere Di-n-butylether, Valeronitril, n-Octan, Cyclooctan, n-Hexylamin, Hexamethylenimin, Hexamethylendiamin bevorzugt Hexamethylenimin und/oder Hexamethylendiamin, besonders bevorzugt Hexamethylenimin.

In einer bevorzugten Ausführungsform wählt man als Verbindung B Hexamethylenimin und/oder Hexamethylendiamin, oder, besonders bevorzugt, man gibt keine weitere Verbindung B zu.

Bevorzugt führt man Verbindung B der Kolonne K3 in einer Menge im Bereich von 0,01 bis 50, vorzugsweise von 0,5 bis 10 Gew.-%, bezogen auf den Sumpf II, zu.

In Stufe (e) unterwirft man den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne (K4 in der Zeichnung) einer Destillation unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und der Druck im Bereich von 0,05 bis 1,5 bar liegen unter Erhalt eines Sumpfes IV.

Gewünschtenfalls stattet man die Kolonne mit einer Trennwand im Bereich zwischen Zulauf und Seitenabzug aus (Petlyuk-Kolonne), so daß das gewonnene Hexamethylendiamin im wesentlichen frei ist von Hexamethylenimin und inerten Verbindung(en) B sowie von anderen Leichtsiedern, wobei
man Kopfprodukt KP1 und/oder HMD aus dem Seitenabzug SA1 bei Bedarf der dritten Kolonne zuführt oder gegebenenfalls nur teilweise der dritten Kolonne zuführt und den Rest ausschleust.

In Stufe (f) unterwirft man den Sumpf IV, enthaltend im wesentlichen 6-Aminocapronitril und Adiponitril sowie eventuelle Hochsieder in einer fünften Kolonne (K5 in der Zeichnung) einer Destillation, unter Erhalt von 6-Aminocapronitril mit einer Reinheit von mindestens 95 %, vorzugsweise 99 bis 99,9 %, als Kopfprodukt und eines Seitenabzuges, bestehend im wesentlichen aus Adipodinitril, sowie eines Sumpfes V, der aus Hochsiedern und geringen Mengen Adipodinitril besteht.

Gewünschtenfalls stattet man die Kolonne mit einer Trennwand im Bereich zwischen Zulauf und Seitenabzug aus, so daß das gewonnene Adipodinitril niedrigere Anteile an Hochsiedern enthält, wobei die Destillation bei einer Sumpftemperatur im Bereich von 50 bis 250°C und einem Druck im Bereich von 10 bis 300 mbar durchgeführt wird.

Adipodinitril (ADN) aus Seitenabzug 2 (SA2) wird in den Reaktor R1 zurückgeführt.

Erfindungsgemäß setzt man das erhaltene 6-Aminocapronitril zu Caprolactam um. Diese Cyclisierung kann man nach bekannten Verfahren in der Flüssig- oder Gasphase durchführen, beispielsweise nach einem Verfahren aus der US 2 301 964, US 2 357 484, EP-A 150 295 oder der DE-A 43 19 134, indem man üblicherweise das 6-Aminocapronitril mit Wasser in der Flüssigphase zu Caprolactam und Ammoniak umsetzt.

Bei der Umsetzung ohne Katalysator wählt man eine Temperatur im Bereich von 200 bis 375°C und Reaktionszeiten im Bereich von 10 bis 90, vorzugsweise von 10 bis 30 min. Als Lösungsmittel verwendet man in der Regel Wasser, wobei der 6-Aminocapronitril-Gehalt, bezogen auf das Wasser, im allgemeinen im Bereich von unter 30, vorzugsweise von 10 bis 25 Gew.-% gewählt wird.

Bei der Umsetzung in der Flüssigphase in Gegenwart eines Katalysators wählt man üblicherweise eine Temperatur im Bereich von 50 bis 330°C, eine Wassermenge im Bereich von 1,3 bis 50, bevorzugt von 1,3 bis 30 Mol pro Mol 6-Aminocapronitril und eine Reaktionszeit im Bereich von 10 min bis mehreren Stunden. Bei Verwendung eines organischen Lösungsmittels, insbesondere eines Alkohols, wählt man im allgemeinen eine Wassermenge im Bereich von 1,3 bis 5 Mol pro Mol 6-Aminocapronitril.

Üblicherweise arbeitet man den bei der Cyclisierung erhaltenen Reaktionsaustrag zunächst destillativ auf, wobei Ammoniak, Wasser und gegebenenfalls organisches Lösungsmittel abgetrennt werden. Der im Sumpf vorhandene Katalysator, falls eingesetzt, wird in der Regel vom Caprolactam nach einer der üblichen Methoden abgetrennt und in den Cyclisierungsreaktor (R2 in der Zeichnung) zurückgeführt. Das Rohcaprolactam wird im allgemeinen durch an sich bekannte Reinigungsoperationen wie Destillation in Reinlactam überführt, das anschließend zur Polymerisation zu Polycaprolactam zur Verfügung steht.

In einer bevorzugten Ausführungsform setzt man 6-Aminocapronitril mit Wasser in flüssiger Phase unter Verwendung heterogener Katalysatoren um.

Die Umsetzung wird in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol 6-Aminocapronsäurenitril werden im allgemeinen mindestens 0,01 mol, vorzugsweise 0,1 bis 20 und insbesondere 1 bis 5 mol Wasser eingesetzt.

Vorteilhaft wird das 6-Aminocapronsäurenitril in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Wasser/ Lösungsmittel-Gemischen eingesetzt. Als Lösungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, das 6-Aminocapronsäurenitril als Reaktand und gleichzeitig Lösungsmittel anzuwenden.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinnoxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:
Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische Ionenaustauscher wie beispielsweise Naphion® als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren können je nach der Zusammensetzung des Katalysators als Vollkontakt oder Trägerkatalysator verwendet werden. So kann z.B. Titandioxid als Titandioxid-Strang oder als auf einen Träger in dünner Schicht aufgebrachtes Titandioxid eingesetzt werden. Zum Aufbringen von Titandioxid auf einen Träger wie Siliciumdioxid, Aluminiumoxid oder Zirkondioxid sind alle in der Literatur beschriebenen Methoden verwendbar. So kann eine dünne Titandioxid-Schicht durch Hydrolyse von Ti-Organylen wie Ti-Isopropylat oder Ti-Butylat, oder durch Hydrolyse von TiCl₄ oder anderen anorganischen Ti-haltigen Verbindungen aufgebracht werden. Auch Titandioxid-haltige Sole sind verwendbar.

Weitere geeignete Verbindungen sind Zirkonylchlorid, Aluminiumnitrat und Cernitrat.

Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

In einer weiteren bevorzugten Ausführungsform cyclisiert man 6-Aminocapronsäurenitril in Flüssigphase mit Wasser bei erhöhter Temperatur ohne Katalysator, indem man eine wäßrige Lösung von 6-Aminocapronsäurenitril in flüssiger Phase ohne Zusatz eines Katalysators in einem Reaktor erhitzt unter Erhalt einer Mischung I, bestehend aus im wesentlichen Wasser, Caprolactam und einer hochsiedenden Fraktion ("Hochsieder"). In dieser bevorzugten Ausführungsform setzt man Wasser bevorzugt im Überschuß ein, besonders bevorzugt verwendet man je mol 6-Aminocapronsäurenitril 10 bis 150, insbesondere 20 bis 100 mol Wasser unter Erhalt einer wäßrigen Lösung von 6-Aminocapronsäurenitril. In einer weiteren bevorzugten Ausführungsform verwendet man üblicherweise 5 bis 25 mol Wasser je mol 6-Aminocapronsäurenitril und kann die Lösung im allgemeinen durch Zusatz eines organischen Lösungsmittels auf 5 bis 25 Gew.-% 6-Aminocapronsäurenitril weiter verdünnen.

Als geeignete Lösungsmittel seien beispielsweise genannt:
C₁-C₄-Alkanole wie Methanol, Ethanol, n-, i-Propanol, Butanole, Glykole wie Ethylenglykol, Diethylenglykol, Triethylenglykol, Tetraethylenglykol, Ether wie Methyl-tert.-butylether, Diethylenglykoldiethylether, C₆-C₁₀-Alkane wie n-Hexan, n-Heptan, n-Octan, n-Nonan, n-Decan sowie Cyclohexan, Benzol, Toluol, Xylol, Lactame wie Pyrrolidon, Caprolactam oder N-C₁-C₄-Alkyl-Lactame wie N-Methylpyrrolidon, N-Methylcaprolactam oder N-Ethylcaprolactam.

In einer weiteren Ausführungsform kann man dem Reaktionsgemisch von 0 bis 5, bevorzugt von 0,1 bis 2 Gew.-% Ammoniak, Wasserstoff oder Stickstoff zusetzen.

Bevorzugt führt man die Reaktion bei einer Temperatur im Bereich von 200 bis 370, vorzugsweise von 220 bis 350°C, besonders bevorzugt von 240 bis 320°C durch.

Üblicherweise führt man die Reaktion unter Druck durch, wobei man den Druck in der Regel im Bereich von 0,1 bis 50, bevorzugt von 5 bis 25 MPa so wählt, daß das Reaktionsgemisch bevorzugt in flüssiger Phase vorliegt.

Die Reaktionsdauer hängt im wesentlichen von den gewählten Verfahrensparametern ab und liegt beim kontinuierlich durchgeführten Verfahren im allgemeinen im Bereich von 20 bis 180, bevorzugt von 20 bis 90 min. Bei kürzeren Reaktionszeiten sinkt in der Regel der Umsatz, bei längeren Reaktionszeiten bilden sich nach den bisherigen Beobachtungen störende Oligomere.

Die Cyclisierung führt man bevorzugt kontinuierlich, vorzugsweise in einem Rohrreaktor, in Rührkesseln oder Kombinationen davon durch.

Die Cyclisierung kann man auch diskontinuierlich durchführen. Die Reaktionsdauer liegt dann üblicherweise im Bereich von 30 bis 180 min.

Der Austrag ist in der Regel eine Mischung, bestehend im wesentlichen aus 50 bis 98, vorzugsweise von 80 bis 95 Gew.-% Wasser und von 2 bis 50, vorzugsweise von 5 bis 20 Gew.-% einer Mischung, bestehend im wesentlichen aus von 50 bis 90, vorzugsweise von 65 bis 85 Gew.-% Caprolactam und von 10 bis 50, vorzugsweise von 15 bis 35 Gew.-% einer hochsiedenden Fraktion ("Hochsieder").

In einer weiteren Ausführungsform entfernt man nach der partiellen Hydrierung und nach der Abtrennung von Ammoniak und inerter Verbindung(en) A (Sumpfprodukt der Kolonne 3) gegebenenfalls vorhandenen Katalysatorabrieb und Hochsieder durch Verdampfung oder Destillation, indem die nicht gewünschten Stoffe als Sumpf erhalten, und das Kopfprodukt in die Kolonne K4 geführt wird.

In einer weiteren Ausführungsform zieht man Adipodinitril und Hochsieder über Sumpf der Kolonne K5 ab und führt es Stufe (a) zu. Es ist weiterhin in diesem Fall möglich, einen Teilstrom aus dem Sumpf der Kolonne K5 auszuschleusen.

Es besteht auch die Möglichkeit, ADN aus dem Seitenabzug der Kolonne K5 in einer weiteren Kolonne zu reinigen.

Das erfindungsgemäß erhaltene Hexamethylendiamin kann man nach üblichen Methoden weiterreinigen und zur Herstellung von Poly- und Copolymeren wie Polyamid-66 einsetzen.

Erfindungsgemäß kann man einen Teil des Verfahrens zur Herstellung von Caprolactam aus Adipodinitril auch zur gleichzeitigen Trennung von 6-Aminocapronitril und Hexamethylendiamin durch Destillation einer im wesentlichen diese Verbindungen enthaltenden Mischung einsetzen, indem man
(a) eine Mischung, enthaltend im wesentlichen 6-Aminocapronitril, Hexamethylendiamin, Ammoniak, Adipodinitril und Hexamethylenimin einer Destillation unter Erhalt von Ammoniak als Kopfprodukt und eines Sumpfes I unterwirft, wobei man die Destillation bei einer Sumpftemperatur im Bereich von 60 bis 250°C und einem Druck im Bereich von 5 bis 30 bar in Gegenwart von einer unter den Destillationsbedingungen inerten Verbindung oder einem Gemisch von inerten Verbindungen A durchführt, die bei einem Druck von 18 bar bei einer Temperatur im Bereich von 60 bis 250°C siedet, und wobei man den Ammoniak nicht vollständig abtrennt, und
(b) den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber demjenigen aus der Mischung, die in Stufe (a) eingesetzt wird, einer zweiten Destillation unterwirft unter Erhalt einer Mischung aus der inerten Verbindung A und Ammoniak als Kopfprodukt und eines Sumpfes II,
   wobei man die Destillation bei einer Sumpftemperatur im Bereich von 100 bis 250°C, und einem Druck im Bereich von 2 bis 15 bar durchführt, mit der Maßgabe, daß man die Drücke der ersten und der zweiten Kolonne so aufeinander abstimmt, daß man bei einer Sumpftemperatur von jeweils maximal 220°C eine Kopftemperatur von über 20°C erhält,
   die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
   den Kopfabzug der zweiten Kolonne dampfförmig nach Druckerhöhung mit einem Verdichter in die erste Kolonne oder in deren Kondensator zurückführt, und
(c) den Sumpf II, enthaltend im wesentlichen 6-Aminocapronitril, Hexamethylendiamin, Adipodinitril, Hexamethylenimin und inerte Verbindung(en) A einer dritten Kolonne einer Destillation unterwirft unter Erhalt der inerten Verbindung(en) A als Kopfprodukt und eines Sumpfes III, wobei man die Destillation bei einer Sumpftemperatur im Bereich von 50 bis 250°C und einem Druck im Bereich von 0,05 bis 2 bar durchführt, mit der Maßgabe, daß man die als Kopfprodukt erhaltenen inerten Verbindung(en) A der zweiten Kolonne zuführt, und gewünschtenfalls die Destillation in Gegenwart von einer oder mehreren unter den Destillationsbedingungen inerten Verbindung(en) B durchführt, die bei einem Druck von 0,3 bar im Bereich von 20 bis 250°C sieden,
(d) den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne einer Destillation unterwirft unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und einem Druck im Bereich von 0,05 bis 1,5 bar liegen unter Erhalt eines Sumpfes IV, und
   gewünschtenfalls die Kolonne mit einer Trennwand im Bereich zwischen Zulauf und Seitenabzug ausstattet (Petlyuk-Kolonne), so daß das gewonne Hexamethylendiamin im wesentlichen frei ist von Hexamethylenimin und inerten Verbindungen(en) B sowie von anderen Leichtsiedern, wobei
   man Kopfprodukt KP1 der dritten Kolonne zuführt oder gegebenenfalls nur teilweise der dritten Kolonne zuführt und den Rest ausschleust, und
(e) den Sumpf IV, enthaltend im wesentlichen 6-Aminocapronitril und Adipodinitril sowie eventuelle Hochsieder in einer fünften Kolonne einer Destillation unterwirft, unter Erhalt von 6-Aminocapronitril mit einer Reinheit von mindestens 95% als Kopfprodukt und eines Seitenabzuges, bestehend im wesentlichen aus Adipodinitril sowie eines Sumpfes V, das aus Hochsiedern und geringen Mengen Adipodinitril besteht, und gewünschtenfalls die Kolonne mit einer Trennwand im Bereich zwischen Zulauf und Seitenabzug ausstattet, so daß das gewonnene Adipodinitril niedrigere Anteile an Hochsiedern enthält, wobei
   die Destillation bei einer Sumpftemperatur im Bereich von 50 bis 250°C und einem Druck im Bereich von 10 bis 300 mbar durchgeführt wird.

Das erfindungsgemäße Verfahren hat den Vorteil, daß ausgehend von Adipodinitril ein kontinuierliches Verfahren zu Caprolactam zugänglich ist, unter gleichzeitiger Gewinnung von Hexamethylendiamin.

### Beispiele

(a) Hydrierung von Adipodinitril zu 6-Aminocapronitril
   Ein Rohrreaktor von 2 m Länge und 2,5 cm Innendurchmesser wurde mit 750 ml (1534 g) Katalysator, bestehend aus 90 Gew.-% CoO, 5 Gew.-% Mn₂O₃, 3 Gew.-% P₂O₅ und 2 Gew.-% Na₂O, befüllt, und der Katalysator wurde anschließend innerhalb von 48 h in einem Wasserstoffstrom (500 l/h) durch Erhöhung der Temperatur von 30°C auf 280°C drucklos aktiviert. Nach Absenkung der Temperatur auf 45°C (Eingang) bzw. 85°C (Ausgang) wurde dem Reaktor bei 200 bar ein Gemisch aus 380 g/h Adipodintril, 380 g/h Ammoniak und 500 l/h Wasserstoff zugeführt. Zusätzlich wurde zur Wärmeabfuhr die vierfache Zulaufmenge (ca. 3 kg/h) im Kreis gefahren. Das Adipodinitril setzte sich unter diesen Bedingungen zu 70 % um. Das Reaktionsgemisch bestand aus 50 Gew.-% Ammoniak, 15 Gew.-% ADN, 17,5 Gew.-% Aminocapronitril (ACN), 17,4 Gew.-% HMD und 0,1 Gew.-% Sonstige (ACN-Selektivität: 50 %, ACN+HMD-Selektivität: > 99 %).
(b) Destillative Aufarbeitung des Hydrieraustrags
   Der vom Katalysator befreite Hydrieraustrag aus (a) wurde auf den Kopf einer ersten Kolonne mit zwei theoretischen Trennstufen gegeben. Über Kopf wurden bei 47°C und 19 bar 5,0 kg Ammoniak mit 200 ppm Ethanol abgetrennt und zur Hydrierung (Stufe (a)) eingesetzt.
   Der Sumpf der ersten Kolonne, ein Ethanol und geringe Mengen Ammoniak enthaltendes Reaktionsgemisch, wurde bei einer Sumpftemperatur von 180°C in eine zweite Kolonne mit 10 theoretischen Trennstufen gefahren.
   Über Kopf wurden aus dieser Kolonne bei 50°C und 10 bar 1,22 kg eines Gemisches aus 30 Gew.-% Ammoniak und 70 Gew.-% Ethanol sowie Spuren an Hexamethylenimin (HMI), Hexamethylendiamin (HMD), ACN und ADN in die erste Kolonne zurückgeführt.
   Das Sumpfprodukt der zweiten Kolonne, das 39 % Ethanol und 100 ppm Ammoniak enthielt und eine Temperatur von 177°C besaß, wurde auf eine dritte Kolonne mit 10 theoretischen Trennstufen gefahren. Über Kopf wurden aus dieser Kolonne bei 47°C/300 mbar 3,2 kg Ethanol abgezogen und in die zweite Kolonne zurückgeführt.
   Aus dem Sumpf der dritten Kolonne, der eine Temperatur von 166°C aufwies, wurden 5,0 kg Produkt auf eine vierte Kolonne mit 35 theoretischen Trennstufen gegeben. Über Kopf dieser Kolonne wurden bei 67°C/90 mbar 5 g HMI mit 19 % HMD abgezogen. An einem flüssigen Seitenabzug aus der 30, theoretischen Trennstufe dieser Kolonne wurden 1,74 kg HMD mit 155 ppm HMI und 100 ppm ACN abgezogen und aus dem Verfahren ausgeschleust.
   Aus dem Sumpf der vierten Kolonne, der eine Temperatur von 181°C aufwies, wurden 3,25 kg Produkt, bestehend im wesentlichen aus ACN und ADN, ausgeschleust und in eine fünfte Kolonne mit 20 theoretischen Trennstufen gegeben. Über Kopf dieser Kolonne wurden bei 20 mbar und 120°C 1,75 kg ACN mit 185 ppm HMD und 100 ppm ADN gewonnen.
   An einem dampfförmigen Seitenabzug im unteren Teil der fünften Kolonne wurden 1,39 kg ADN mit 800 ppm ACN abgezogen. Über Sumpf wurden 0,11 kg ADN mit ca. 10 Gew.-% Hochsiedern bei einer Temperatur von 190°C abgezogen.
(c) Cyclisierung von 6-Aminocapronitril zu Caprolactam
   Eine Lösung aus 2 kg ACN (aus (b)), 0,64 kg Wasser und 17,4 kg Ethanol wurde bei 220°C und 70 bar mit einer Verweilzeit von 15 min durch einen ölbeheizten, mit Titandioxid-Strängen (4 mm) gefüllten Rohrreaktor (Verhältnis von Länge zu Durchmesser = 100) gefahren. Der Reaktionsaustrag enthielt 1,7 kg Caprolactam, 0,06 kg 6-Aminocapronsäureethylester, 0,1 kg 6-Aminocapronitril (gaschromatographisch bestimmt) sowie 0,1 kg 6-Aminocapronsäure und Oligomere bzw. Polymere des Caprolactams (bestimmt mittels HPLC). Durch fraktionierende Destillation wurden daraus 1,6 kg Caprolactam erhalten.

## Patentansprüche

1. Verfahren zur gleichzeitigen Herstellung von Caprolactam und Hexamethylendiamin ausgehend von Adipodinitril, dadurch gekennzeichnet, daß man
(a) Adipodinitril partiell hydriert unter Erhalt einer Mischung, enthaltend im wesentlichen 6-Aminocapronitril, Hexamethylendiamin, Ammoniak, Adipodinitril und Hexamethylenimin, und
(b) die in (a) erhaltene Mischung einer Destillation unter Erhalt von Ammoniak als Kopfprodukt und eines Sumpfes I unterwirft, wobei man die Destillation bei einer Sumpftemperatur im Bereich von 60 bis 250°C und einem Druck im Bereich von 5 bis 30 bar in Gegenwart von einer unter den Destillationsbedingungen inerten Verbindung A oder einem Gemisch von inerten Verbindungen A durchführt, die bei einem Druck von 18 bar bei einer Temperatur im Bereich von 60 bis 250°C sieden, und wobei man den Ammoniak nicht vollständig abtrennt, und
(c) den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber demjenigen aus der Mischung, die in Stufe (b) eingesetzt wird, einer zweiten Destillation unterwirft unter Erhalt einer Mischung aus den inerten Verbindung(en) A und Ammoniak als Kopfprodukt und eines Sumpfes II, wobei man die Destillation bei einer Sumpftemperatur im Bereich von 100 bis 250°C, und einem Druck im Bereich von 2 bis 15 bar durchführt, mit der Maßgabe, daß man die Drücke der ersten und der zweiten Kolonne so aufeinander abstimmt, daß man bei einer jeweiligen Sumpftemperatur von maximal 250°C eine Kopftemperatur von über 20°C erhält, oder
die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
den Kopfabzug der zweiten Kolonne dampfförmig nach Druckerhöhung mit einem Verdichter in die erste Kolonne oder in deren Kondensator zurückführt und
(d) den Sumpf II, enthaltend im wesentlichen 6-Aminocapronitril, Hexamethylendiamin, Adipodinitril, Hexamethylenimin und inerte Verbindung(en) A in einer dritten Kolonne einer Destillation unterwirft unter Erhalt der inerten Verbindung(en) A als Kopfprodukt und eines Sumpfes III, wobei man die Destillation bei einer Sumpftemperatur im Bereich von 50 bis 250°C und einem Druck im Bereich von 0,05 bis 2 bar durchführt, mit der Maßgabe, daß man die als Kopfprodukt erhaltenen inerten Verbindungen A der zweiten Kolonne zuführt, und gewünschtenfalls die Destillation in Gegenwart von einer oder mehreren unter den Destillationsbedingungen inerten Verbindung(en) B durchführt, die bei einem Druck von 0,3 bar im Bereich von 20 bis 250°C sieden,
(e) den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne einer Destillation unterwirft unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und einem Druck im Bereich von 0,05 bis 1,5 bar liegen unter Erhalt eines Sumpfes IV, und
gewünschtenfalls die Kolonne mit einer Trennwand im Bereich zwischen Zulauf und Seitenabzug ausstattet (Petlyuk-Kolonne), so daß das gewonnene Hexamethylendiamin im wesentlichen frei ist von Hexamethylenimin und inerten Verbindung(en) B sowie von anderen Leichtsiedern, wobei
man Kopfprodukt KP1 der dritten Kolonne zuführt oder gegebenenfalls nur teilweise der dritten Kolonne zuführt und den Rest ausschleust, und
(f) den Sumpf IV, enthaltend im wesentlichen 6-Aminocapronitril und Adipodinitril sowie eventuelle Hochsieder in einer fünften Kolonne einer Destillation unterwirft, unter Erhalt von 6-Aminocapronitril mit einer Reinheit von mindestens 95% als Kopfprodukt und eines Seitenabzuges, bestehend im wesentlichen aus Adipodinitril, sowie eines Sumpfes V, der aus Hochsiedern und geringen Mengen Adipodinitril besteht, und
gewünschtenfalls die Kolonne mit einer Trennwand im Bereich zwischen Zulauf und Seitenabzug ausstattet, so daß das gewonnene Adipodinitril niedrigere Anteile an Hochsiedern enthält, wobei
die Destillation bei einer Sumpftemperatur im Bereich von 50 bis 250°C und einem Druck im Bereich von 10 bis 300 mbar durchgeführt wird,
und das so erhaltene 6-Aminocapronitril anschließend zu Caprolactam cyclisiert.

2. Verfahren gemäß Anspruch 1, dadurch gekennzeichnet, daß man das Adipodinitril aus dem Seitenabzug von Kolonne 5 in Stufe (a) zuführt.

3. Verfahren zur gleichzeitigen Trennung von 6-Aminocapronitril und Hexamethylendiamin durch Destillation einer im wesentlichen diese Verbindungen enthaltenden Mischung, dadurch gekennzeichnet, daß man
(a) eine Mischung, enthaltend im wesentlichen 6-Aminocapronitril, Hexamethylendiamin, Ammoniak, Adipodinitril und Hexamethylenimin einer Destillation unter Erhalt von Ammoniak als Kopfprodukt und eines Sumpfes I unterwirft, wobei man die Destillation bei einer Sumpftemperatur im Bereich von 60 bis 250°C und einem Druck im Bereich von 5 bis 30 bar in Gegenwart von einer unter den Destillationsbedingungen inerten Verbindung A oder einem Gemisch von inerten Verbindungen A durchführt, die bei einem Druck von 18 bar bei einer Temperatur im Bereich von 60 bis 250°C sieden, und wobei man den Ammoniak nicht vollständig abtrennt, und
(b) den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber demjenigen aus der Mischung, die in Stufe (a) eingesetzt wird, einer zweiten Destillation unterwirft unter Erhalt einer Mischung aus der inerten Verbindung A und Ammoniak als Kopfprodukt und eines Sumpfes II, wobei man die Destillation bei einer Sumpftemperatur im Bereich von 100 bis 250°C, und einem Druck im Bereich von 2 bis 15 bar durchführt, mit der Maßgabe, daß man die Drücke der ersten und der zweiten Kolonne so aufeinander abstimmt, daß man bei einer Sumpftemperatur von jeweils maximal 250°C eine Kopftemperatur von über 20°C erhält, oder
die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
den Kopfabzug der zweiten Kolonne dampfförmig nach Druckerhöhung mit einem Verdichter in die erste Kolonne oder in deren Kondensator zurückführt und
(c) den Sumpf II, enthaltend im wesentlichen 6-Aminocapronitril, Hexamethylendiamin, Adipodinitril, Hexamethylenimin und inerte Verbindung(en) A einer dritten Kolonne einer Destillation unterwirft unter Erhalt der inerten Verbindung(en) A als Kopfprodukt und eines Sumpfes III, wobei man die Destillation bei einer Sumpftemperatur im Bereich von 50 bis 250°C und einem Druck im Bereich von 0,05 bis 2 bar durchführt, mit der Maßgabe, daß man die als Kopfprodukt erhaltenen inerten Verbindung(en) A der zweiten Kolonne zuführt, und gewünschtenfalls die Destillation in Gegenwart von einer oder mehreren unter den Destillationsbedingungen inerten Verbindungen B durchführt, die bei einem Druck von 0,3 bar im Bereich von 20 bis 250°C sieden,
(d) den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne einer Destillation unterwirft unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und der Druck im Bereich von 0,05 bis 1,5 bar liegen unter Erhalt eines Sumpfes IV, und
gewünschtenfalls die Kolonne mit einer Trennwand im Bereich zwischen Zulauf und Seitenabzug ausstattet (Petlyuk-Kolonne), so daß das gewonnene Hexamethylendiamin im wesentlichen frei ist von Hexamethylenimin und inerten Verbindung(en) B sowie von anderen Leichtsiedern, wobei
man Kopfprodukt KP1 der dritten Kolonne zuführt oder gegebenenfalls nur teilweise der dritten Kolonne zuführt und den Rest ausschleust, und
(e) den Sumpf IV, enthaltend im wesentlichen 6-Aminocapronitril und Adipodinitril sowie eventuelle Hochsieder in einer fünften Kolonne einer Destillation unterwirft, unter Erhalt von 6-Aminocapronitril mit einer Reinheit von mindestens 95% als Kopfprodukt und eines Seitenabzuges, bestehend im wesentlichen aus Adipodinitril, sowie eines Sumpfes V, das aus Hochsiedern und geringen Mengen Adipodinitril besteht, und
gewünschtenfalls die Kolonne mit einer Trennwand im Bereich zwischen Zulauf und Seitenabzug ausstattet, so daß das gewonnene Adipodinitril niedrigere Anteile an Hochsiedern enthält, wobei
die Destillation bei einer Sumpftemperatur im Bereich von 50 bis 250°C und einem Druck im Bereich von 10 bis 300 mbar durchgeführt wird.

4. Verfahren nach einem der Ansprüche 1 oder 2, dadurch gekennzeichnet, daß man in Stufe (b) eine Mischung einsetzt, bestehend im wesentlichen aus
von 1 bis 70 Gew.-% 6-Aminocapronitril,
von 1 bis 70 Gew.-% Adipodinitril,
von 0,1 bis 70 Gew.-% Hexamethylendiamin,
von 0,01 bis 10 Gew.-% Hexamethylenimin und
von 5 bis 95 Gew.-% Ammoniak.

5. Verfahren nach Anspruch 3, dadurch gekennzeichnet, daß man in Stufe (a) eine Mischung einsetzt, bestehend im wesentlichen aus
von 1 bis 70 Gew.-% 6-Aminocapronitril,
von 1 bis 70 Gew.-% Adipodinitril,
von 0,1 bis 70 Gew.-% Hexamethylendiamin,
von 0,01 bis 10 Gew.-% Hexamethylenimin und
von 5 bis 95 Gew.-% Ammoniak.

6. Verfahren nach den Ansprüchen 1 bis 5, dadurch gekennzeichnet, daß man als inerte Verbindung A Ethanol einsetzt.

7. Verfahren nach den Ansprüchen 1 bis 6, dadurch gekennzeichnet, daß man als Komponente B Hexamethylenimin und/oder Hexamethylendiamin einsetzt.

8. Verfahren nach Anspruch 3, dadurch gekennzeichnet, daß man als inerte Verbindung A Ethanol einsetzt.

9. Verfahren nach Anspruch 3, dadurch gekennzeichnet, daß man als Komponente B Hexamethylenimin und/oder Hexamethylendiamin einsetzt.

## Claims

1. A process for the simultaneous preparation of caprolactam and hexamethylenediamine starting from adiponitrile, wherein
(a) adiponitrile is partially hydrogenated to give a mixture containing essentially 6-aminocapronitrile, hexamethylenediamine, ammonia, adiponitrile and hexamethyleneimine, and
(b) the mixture obtained in (a) is subjected to a distillation to give ammonia as the top product and a bottom product I, the distillation being carried out at a bottom temperature of from 60 to 250°C and a pressure of from 5 to 30 bar in the presence of a compound A which is inert under the distillation conditions, or a mixture of inert compounds A, which boils at from 60 to 250°C at 18 bar, and the ammonia not being completely separated off, and
(c) the bottom product I, containing essentially
6-aminocapronitrile, hexamethylenediamine, adiponitrile, hexamethyleneimine, inert compound or compounds A and ammonia, the ammonia content being lower than that of the mixture used in stage (b), is subjected to a second distillation to give a mixture of the inert compound or compounds A and ammonia as the top product and a bottom product II, the distillation being carried out at a bottom temperature of from 100 to 250°C and a pressure of from 2 to 15 bar, with the proviso that the pressures of the first and of the second column are matched with one another so that, at a bottom temperature of not more than 250°C in each case, a top temperature of more than 20°C is obtained, or
the condensation at the top of the second column is carried out at lower temperatures, the top product, which consists of pure or relatively highly concentrated ammonia, being recycled to the first column, or
the top product of the second column is recycled to the first column or to the condenser thereof in vapor form after the pressure has been increased by means of a compressor,
(d) the bottom product II, containing essentially
6-aminocapronitrile, hexamethylenediamine, adiponitrile, hexamethyleneimine and inert compound or compounds A, is subjected to a distillation in a third column to give the inert compound or compounds A as the top product and a bottom product III, the distillation being carried out at a bottom temperature of from 50 to 250°C and a pressure of from 0.05 to 2 bar, with the proviso that the inert compounds A obtained as the top product are fed to the second column, and, if desired, the distillation being carried out in the presence of one or more compounds B which are inert under the distillation conditions and boil at from 20 to 250°C at 0.3 bar,
(e) the bottom product III, containing essentially
6-aminocapronitrile, hexamethylenediamine, adiponitrile, hexamethyleneimine and, if desired, inert compound or compounds B, is subjected to distillation in a fourth column to give a top product KP1, containing essentially hexamethyleneimine, if desired inert compound or compounds B, and a side stream SA1, containing essentially hexamethylenediamine, the bottom temperature of the column being from 50 to 250°C and the pressure from 0.05 to 1.5 bar, and to give a bottom product IV, and
if desired, the column is equipped with a dividing wall in the region between feed and side take-off point (Petlyuk column), so that the hexamethylenediamine obtained is essentially free of hexamethyleneimine and inert compound or compounds B and of other low boilers,
top product KP1 being fed to the third column or, if required, only some of top product KP1 being fed to the third column and the remainder being removed, and
(f) the bottom product IV, containing essentially
6-aminocapronitrile and adiponitrile and possibly high boilers, is subjected to a distillation in a fifth column to give 6-aminocapronitrile having a purity of at least 95% as the top product and a side stream consisting essentially of adiponitrile and a bottom product V which consists of high boilers and small amounts of adiponitrile, and,
if desired, the column is equipped with a dividing wall in the region between feed and side take-off point, so that the adiponitrile obtained contains relatively small amounts of high boilers,
the distillation being carried out at a bottom temperature of from 50 to 250°C and a pressure of from 10 to 300 mbar,
and the resulting 6-aminocapronitrile is then cyclized to caprolactam.

2. A process as claimed in claim 1, wherein the adiponitrile from the side stream of column 5 is fed to stage (a).

3. A process for the simultaneous separation of
6-aminocapronitrile and hexamethylenediamine by distillation of a mixture containing essentially these compounds, wherein
(a) a mixture containing essentially 6-aminocapronitrile, hexamethylenediamine, ammonia, adiponitrile and hexamethyleneimine is subjected to a distillation to give ammonia as the top product and a bottom product I, the distillation being carried out at a bottom temperature of from 60 to 250°C and at from 5 to 30 bar in the presence of a compound A which is inert under the distillation conditions, or a mixture of inert compounds A, which boil at from 60 to 250°C at 18 bar, and the ammonia not being completely separated off, and
(b) the bottom product I, containing essentially
6-aminocapronitrile, hexamethylenediamine, adiponitrile, hexamethyleneimine, inert compound or compounds A and ammonia, the ammonia content being lower than that of the mixture used in stage (a), is subjected to a second distillation to give a mixture of the inert compound A and ammonia as the top product and a bottom product II, the distillation being carried out at a bottom temperature of from 100 to 250°C and a pressure of from 2 to 15 bar, with the proviso that the pressures of the first and of the second column are matched with one another so that, at a bottom temperature of not more than 250°C in each case, a top temperature of more than 20°C is obtained, or,
the condensation at the top of the second column is carried out at lower temperatures, the top product, which consists of pure or relatively highly concentrated ammonia, being recycled to the first column, or
the top product of the second column is recycled to the first column or to the condenser thereof in vapor form after the pressure has been increased by means of a compressor,
(c) the bottom product II, containing essentially
6-aminocapronitrile, hexamethylenediamine, adiponitrile, hexamethyleneimine and inert compound or compounds A, is subjected to a distillation in a third column to give the inert compound or compounds A as the top product and a bottom product III, the distillation being carried out at a bottom temperature of from 50 to 250°C and a pressure of from 0.05 to 2 bar, with the proviso that the inert compound or compounds A obtained as the top product are fed to the second column, and, if desired, the distillation being carried out in the presence of one or more compounds B which are inert under the distillation conditions and boil at from 20 to 250°C at 0.3 bar,
(d) the bottom product III, containing essentially
6-aminocapronitrile, hexamethylenediamine, adiponitrile, hexamethyleneimine and, if desired, inert compound or compounds B, is subjected to distillation in a fourth column to give a top product KP1, containing essentially hexamethyleneimine, if desired inert compound or compounds B, and a side stream SA1, containing essentially hexamethylenediamine, the bottom temperature of the column being from 50 to 250°C and the pressure from 0.05 to 1.5 bar, and to give a bottom product IV, and,
if desired, the column is equipped with a dividing wall in the region between feed and side take-off point (Petlyuk column), so that the hexamethylenediamine obtained is essentially free of hexamethyleneimine and inert compound or compounds B and of other low boilers,
top product KP1 being fed to the third column or, if required, only some of top product KP1 being fed to the third column and the remainder being removed, and
(e) the bottom product IV, containing essentially
6-aminocapronitrile and adiponitrile and possibly high boilers, is subjected to a distillation in a fifth column to give 6-aminocapronitrile having a purity of at least 95% as the top product and a side stream consisting essentially of adiponitrile and a bottom product V which consists of high boilers and small amounts of adiponitrile, and,
if desired, the column is equipped with a dividing wall in the region between feed and side take-off point, so that the adiponitrile obtained contains relatively small amounts of high boilers,
the distillation being carried out at a bottom temperature of from 50 to 250°C and at from 10 to 300 mbar.

4. A process as claimed in either of claims 1 and 2, wherein a mixture consisting essentially of
from 1 to 70% by weight of 6-aminocapronitrile,
from 1 to 70% by weight of adiponitrile,
from 0.1 to 70% by weight of hexamethylenediamine,
from 0.01 to 10% by weight of hexamethyleneimine and
from 5 to 95% by weight of ammonia,
is used in stage (b).

5. A process as claimed in claim 3, wherein a mixture consisting essentially of
from 1 to 70% by weight of 6-aminocapronitrile,
from 1 to 70% by weight of adiponitrile,
from 0.1 to 70% by weight of hexamethylenediamine,
from 0.01 to 10% by weight of hexamethyleneimine and
from 5 to 95% by weight of ammonia,
is used in stage (a).

6. A process as claimed in any of claims 1 to 5, wherein ethanol is used as the inert compound A.

7. A process as claimed in any of claims 1 to 6, wherein hexamethyleneimine or hexamethylenediamine is used as component B.

8. A process as claimed in claim 3, wherein ethanol is used as the inert compound A.

9. A process as claimed in claim 3, wherein hexamethyleneimine or hexamethylenediamine is used as component B.

## Revendications

1. Procédé de préparation simultanée de caprolactame et d'hexaméthylènediamine à partir d'adipodinitrile, caractérisé en ce que
(a) on hydrogène partiellement de l'adipodinitrile avec obtention d'un mélange, contenant essentiellement du 6-aminocapronitrile, de l'hexaméthylènediamine, de l'ammoniac, de l'adipodinitrile et de l'hexaméthylèneimine, et
(b) on soumet le mélange obtenu sous (a) à une distillation avec obtention d'ammoniac, comme produit de tête, et d'un fond de cuve I, la distillation étant effectuée à une température de fond de cuve de l'ordre de 60 à 250°C et à une pression de l'ordre de 5 à 30 bars en présence d'un composé A inerte dans les conditions de distillation ou d'un mélange de composés A inertes, qui bouillent à une pression de 18 bars à une température de l'ordre de 60 à 250°C, l'ammoniac n'étant pas totalement séparé, et
(c) on soumet à une deuxième distillation le fond de cuve I, contenant essentiellement du 6-aminocapronitrile, de l'hexaméthylènediamine, de l'adipodinitrile, de l'hexaméthylèneimine, du ou des composés A inertes, ainsi que de l'ammoniac, la teneur en ammoniac étant plus faible que celle du mélange qui est mis en oeuvre dans l'étape (b), avec obtention d'un mélange du ou des composés A inertes et d'ammoniac, comme produit de tête, et d'un fond de cuve II, la distillation étant effectuée à une température de fond de cuve de l'ordre de 100 à 250°C et à une pression de l'ordre de 2 à 15 bars, à la condition qu'on accorde l'une à l'autre les pressions des première et deuxième colonnes de façon à obtenir pour une température de fond de cuve respective d'au maximum 250°C une température de tête supérieure à 20°C ou qu'on effectue la condensation en tête de la deuxième colonne à des températures plus basses, le produit soutiré en tête, qui est constitué d'ammoniac pur ou concentré de manière élevée, étant recyclé dans la première colonne, ou
on recycle le produit soutiré en tête de la deuxième colonne sous forme de vapeur dans la première colonne ou dans son condenseur après augmentation de pression avec un compresseur, et
(d) on soumet à une distillation dans une troisième colonne le fond de cuve II, contenant essentiellement du 6-aminocapronitrile, de l'hexaméthylènediamine, de l'adipodinitrile, de l'hexaméthylèneimine et du ou des composés A inertes, avec obtention du ou des composés A inertes, comme produit de tête, et d'un fond de cuve III, la distillation étant effectuée à une température de fond de cuve de l'ordre de 50 à 250°C et à une pression de l'ordre de 0,05 à 2 bars à la condition qu'on amène les composés inertes A, obtenus comme produit de tête, à la deuxième colonne, la distillation étant éventuellement effectuée en présence d'un ou de plusieurs composés B inertes dans les conditions de distillation, qui bouillent à une pression de 0,3 bar dans la gamme de 20 à 250°C,
(e) on soumet à une distillation dans une quatrième colonne le fond de cuve III, contenant essentiellement du 6-aminocapronitrile, de l'hexaméthylènediamine, de l'adipodinitrile, de l'hexaméthylèneimine et éventuellement un ou des composés B inertes, avec obtention d'un produit de tête KP1, contenant essentiellement de l'hexaméthylèneimine, éventuellement du ou des composés B inertes, et d'un produit de soutirage latéral SA1, contenant essentiellement de l'hexaméthylènediamine, la température de fond de cuve de la colonne étant de l'ordre de 50 à 250°C à une pression de l'ordre de 0,05 à 1,5 bar, avec obtention d'un fond de cuve IV, et
la colonne étant éventuellement équipée d'une paroi séparatrice dans la zone entre l'alimentation et le soutirage latéral (colonne de Petlyuk), de façon que l'hexaméthylènediamine ainsi produite soit sensiblement exempte d'hexaméthylèneimine et du ou des composés B inertes ainsi que d'autres agents à bas point d'ébullition, le produit de tête KP1 étant amené à la troisième colonne ou étant amené éventuellement seulement partiellement à la troisième colonne, le reste étant évacué par éclusage, et
(f) on soumet à une distillation dans une cinquième colonne le fond de cuve IV, contenant essentiellement du 6-aminocapronitrile et de l'adipodinitrile ainsi que d'éventuels agents à point d'ébullition élevé, avec obtention de 6-aminocapronitrile ayant un degré de pureté d'au moins 95%, comme produit de tête, et d'un produit de soutirage latéral, constitué essentiellement d'adipodinitrile, ainsi que d'un fond de cuve V, qui est constitué d'agents à point d'ébullition élevé et de faibles quantités d'adipodinitrile, et
la colonne est éventuellement équipée d'une paroi séparatrice dans la zone entre l'alimentation et le soutirage latéral de façon que l'adipodinitrile obtenu contienne de plus faibles fractions d'agents à point d'ébullition élevé,
la distillation étant effectuée à une température de fond de cuve de l'ordre de 50 à 250°C et à une pression de l'ordre de 10 à 300 mbars,
et on cyclise ensuite le 6-aminocapronitrile ainsi obtenu en caprolactame.

2. Procédé suivant la revendication 1, caractérisé en ce qu'on amène l'adipodinitrile provenant du soutirage latéral de la colonne 5 dans l'étape (a).

3. Procédé de séparation simultanée de 6-aminocapronitrile et d'hexaméthylènediamine par distillation d'un mélange contenant essentiellement ces composés, caractérisé en ce que
(a) on soumet à une distillation un mélange, contenant essentiellement du 6-aminocapronitrile, de l'hexaméthylènediamine, de l'ammoniac, de l'adipodinitrile et de l'hexaméthylèneimine, avec obtention d'ammoniac, comme produit de tête, et d'un fond de cuve I, la distillation étant effectuée à une température de fond de cuve de l'ordre de 60 à 250°C et à une pression de l'ordre de 5 à 30 bars en présence d'un composé A inerte dans les conditions de distillation ou d'un mélange de composés A inertes, qui bouillent à une pression de 18 bars à une température de l'ordre de 60 à 250°C, l'ammoniac n'étant pas totalement isolé, et
(b) on soumet à une deuxième distillation le fond de cuve I contenant essentiellement du 6-aminocapronitrile, de l'hexaméthylènediamine, de l'adipodinitrile, de l'hexaméthylèneimine, du ou des composés A inertes ainsi que de l'ammoniac, la teneur en ammoniac étant plus faible que celle du mélange qui est mis en oeuvre dans l'étape (a), avec obtention d'un mélange de composés A inertes et d'ammoniac, comme produit de tête, et d'un fond de cuve II, la distillation étant effectuée à une température de fond de cuve de l'ordre de 100 à 250°C et à une pression de l'ordre de 2 à 15 bars, avec la condition qu'on accorde l'une à l'autre les pressions des première et deuxième colonnes de façon à obtenir pour une température de fond de cuve de respectivement au maximum 250°C une température de tête supérieure à 20°C, ou
on effectue la condensation à la tête de la deuxième colonne à des températures plus basses, le produit de soutirage de tête, qui est constitué d'ammoniac pur ou d'une concentration élevée, étant recyclé dans la première colonne, ou
on recycle le produit de soutirage de tête de la deuxième colonne sous forme de vapeur dans la première colonne ou dans son condenseur après élévation de pression avec un compresseur, et
(c) on soumet à une distillation dans une troisième colonne le fond de cuve II, contenant essentiellement du 6-aminocapronitrile, de l'hexaméthylènediamine, de l'adipodinitrile, de l'hexaméthylèneimine et du ou des composés A inertes, avec obtention du ou des composés A inertes, comme produit de tête, et d'un fond de cuve III, la distillation étant effectuée à une température de fond de cuve de l'ordre de 50 à 250°C et à une pression de l'ordre de 0,05 à 2 bars à la condition qu'on amène le ou les composés A inertes obtenus, comme produit de tête, à la deuxième colonne, la distillation étant éventuellement effectuée en présence d'un ou de plusieurs composés B inertes dans les conditions de distillation, qui bouillent à une pression de 0,3 bar dans la gamme de 20 à 250°C,
(d) on soumet à une distillation dans une quatrième colonne le fond de cuve III, contenant essentiellement du 6-aminocapronitrile, de l'hexaméthylènediamine, de l'adipodinitrile, de l'hexaméthylèneimine et éventuellement du ou des composés B inertes, avec obtention d'un produit de tête KP1, contenant essentiellement de l'hexaméthylèneimine, éventuellement du ou des composés B inertes, et d'un produit de soutirage latéral SA1, contenant essentiellement de l'hexaméthylènediamine, la température de fond de cuve de la colonne étant de l'ordre de 50 à 250°C et la pression de l'ordre de 0,05 à 1,5 bar, avec obtention d'un fond de cuve IV, et
la colonne est éventuellement équipée d'une paroi séparatrice dans la zone entre l'alimentation et le soutirage latéral (colonne de Petlyuk), de façon que l'hexaméthylènediamine produite soit sensiblement exempte d'hexaméthylèneimine et du ou des composés B inertes ainsi que d'autres agents à bas point d'ébullition, le produit de tête KP1 étant amené à la troisième colonne ou étant amené éventuellement seulement partiellement à la troisième colonne, le reste étant évacué par éclusage, et
(e) on soumet à une distillation dans une cinquième colonne le fond de cuve IV, contenant essentiellement du 6-aminocapronitrile et de l'adipodinitrile ainsi que d'éventuels agents à point d'ébullition élevé, avec obtention de 6-aminocapronitrile ayant un degré de pureté d'au moins 95%, comme produit de tête, et d'un produit de soutirage latéral, constitué essentiellement d'adipodinitrile, ainsi que d'un fond de cuve V, qui est constitué d'agents à point d'ébullition élevé et de faibles quantités d'adipodinitrile, et
la colonne est éventuellement équipée d'une paroi séparatrice dans la zone entre l'alimentation et le soutirage latéral, de façon que l'adipodinitrile obtenu contienne de plus faibles fractions d'agents à point d'ébullition élevé,
la distillation étant effectuée à une température de fond de cuve de l'ordre de 50 à 250°C et à une pression de l'ordre de 10 à 300 mbars.

4. Procédé suivant l'une des revendications 1 et 2, caractérisé en ce qu'on met en oeuvre dans l'étape (b) un mélange constitué essentiellement
de 1 à 70% en poids 6-aminocapronitrile,
de 1 à 70% en poids d'adipodinitrile,
de 0,1 à 70% en poids d'hexaméthylènediamine,
de 0,01 à 10% en poids d'hexaméthylèneimine, et
de 5 à 95% en poids d'ammoniac.

5. Procédé suivant la revendication 3, caractérisé en ce qu'on met en oeuvre dans l'étape (a) un mélange constitué essentiellement
de 1 à 70% en poids 6-aminocapronitrile,
de 1 à 70% en poids d'adipodinitrile,
de 0,1 à 70% en poids d'hexaméthylènediamine,
de 0,01 à 10% en poids d'hexaméthylèneimine, et
de 5 à 95% en poids d'ammoniac.

6. Procédé suivant l'une des revendications 1 à 5, caractérisé en ce que, comme composé A inerte, on met en oeuvre de l'éthanol.

7. Procédé suivant l'une des revendications 1 à 6, caractérisé en ce que, comme composant B, on met en oeuvre de l'hexaméthylèneimine et/ou de l'hexaméthylènediamine.

8. Procédé suivant la revendication 3, caractérisé en ce que, comme composé A inerte, on met en oeuvre de l'éthanol.

9. Procédé suivant la revendication 3, caractérisé en ce que, comme composé B, on met en oeuvre de l'hexaméthylèneimine et/ou de l'hexaméthylènediamine.
